# EUROPEAN PATENT SPECIFICATION

(11) **EP 2 504 309 B1**
(45) Date of publication and mention of the grant of the patent: **06.08.2014**
(21) Application number: 10792866.5
(22) Date of filing: 24.11.2010
(51) Int. Cl.: C07C 67/29, C07C 67/08, C07C 69/003, C07C 69/16, C07D 209/18, C07D 213/55, C07D 215/14, C07D 239/42, C07D 311/96, C07D 405/06, C07D 407/06

(54) **Synthesis of acetoxyacetaldehyde**
Synthese von Acetoxyacetaldehyd
Synthèse d'acétoxyacétaldéhyde

(30) Priority: 25.11.2009 EP 09177008
(43) Date of publication of application: 03.10.2012
(73) Proprietor: LEK Pharmaceuticals d.d., 1526 Ljubljana (SI)
(72) Inventor: UDOVIC, Marko, 1526 Ljubljana (SI); TRAMSEK, Marko, 1526 Ljubljana (SI); PLANTAN, Ivan, 1526 Ljubljana (SI); CLUZEAU, Jerome, 1526 Ljubljana (SI)
(74) Representative: Prüfer & Partner GbR European Patent Attorneys
(86) International application number: PCT/EP2010/068117
(87) International publication number: WO 2011/064249

(56) References cited:
- WO-A2-2009/092702
- US-A1- 2006 122 434
- NORTHRUP A B ET AL: "Two-step synthesis of carbohydrates by selective aldol reactions", SCIENCE, AMERICAN ASSOCIATION FOR THE ADVANCEMENT OF SCIENCE, WASHINGTON, DC; US, vol. 305, 17 January 2004 (2004-01-17), pages 1752-1755, XP002513579, ISSN: 0036-8075 cited in the application & Alan B. Northrup, David W.C. MacMillan: "Two-Step Synthesis of Carbohydrates by Selective Aldol Reactions", Science, 2004, pages S1-S22, Retrieved from the Internet: URL:http://www.sciencemag.org/cgi/data/110 1710/DC1/1 [retrieved on 2010-02-26]
- L. DE LUCA, G. GIACOMELLI, A. PORCHEDDU: "A Very Mild and Chemoselective Oxidation of Alcohols to Carbonyl Compounds", ORGENIC LETTERS, vol. 3, no. 19, 2001, pages 3041-3043, XP002570708,
- P.L. ANELLI ET AL.: "Fast and Selective Oxidation of Primary Alcohols to Aldehydes or to Carboxylic Acids and of Secondary Alcohols to Ketones Mediated by Oxoammonium Salts under Two-Phase Conditions", JOURNAL OF ORGANIC CHEMISTRY, vol. 52, 1987, pages 2559-2562, XP002570709,
- SRINIVAS K V N S ET AL: "SELECTIVE MONOACETYLATION OF SYMMETRICAL DIOLS AND SELECTIVE MONODEACETYLATION OF SYMMETRICAL DIACETATES USING HY-ZEOLITE AS REUSABLE HETEROGENEOUS CATALYST", SYNLETT, GEORG THIEME VERLAG, DE, no. 15, 1 January 2003 (2003-01-01), pages 2419-2421, XP001206271, ISSN: 0936-5214

## Description

### Field of the Invention

The invention belongs to the field of organic chemistry and relates to a new synthesis of statin compounds or pharmaceutically acceptable salts thereof, comprising a process for preparing acetoxyacetaldehyde.

### Background of the Invention

Acetoxyacetaldehyde (2-acetyloxyacetaldehyde, AOA) is a versatile starting material for various synthetic approaches. Although being a very simple molecule it was disclosed relatively late and proved only indirectly via hydrazones (J. Chem. Soc. 1914, 105, 386; Chem. Ber. 1961, 94, 1860; J. Org. Chem. 1962, 27, 2920). In earlier synthetic methods acetyloxy ethens (Bull. Acad. Sci. USSR 1976, 25 1545) and acetals (Liebigs Ann. Chem. 1991, 1013) were used.

Modern approaches which enabled isolation of AOA with less impurities are based on oxidation of (E)-1,4-diacetoxy-2-butene (DACB). For example the mixture of sodium metaperiodate (NalO₄) and osmium (VIII) oxide (OsO₄) can be used (J. Org. Chem. 1981, 46, 1734). The reaction is carried out in presence of water.

Further approach using DACB (Tetrahedron Lett. 1997, 38, 3595) or allyl acetate (J. Nat. Prod. 1995, 58, 1270) includes oxidation by ozone. Moreover, WO 2009/092702 describes the preparation of AOA by ozonolysis of DACB as an intermediate in the production of statins, particularly in the production of rosuvastatin.

Intermediate DACB can be prepared by acetylation of 2-butene-1,4-diol with acetanyhydride (Ac₂O) or acetyl chloride (AcCl) under basic conditions (J. Org. Chem. 1981, 46, 1734). Similar synthetic route for AOA synthesis, which uses 2-butene-1,4-diol as starting material and further NalO₄ as oxidant is also described in WO 08/034598 (Scheme 1).

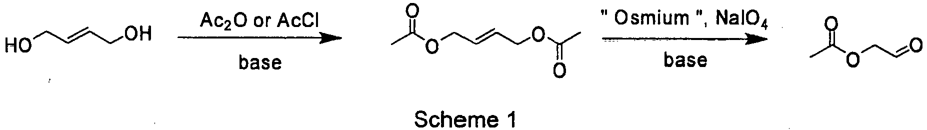

US 2286034 describes the use of monoacetyl ethyleneglycol (MAG) as starting material. The reaction is carried out at high temperatures (200-375 °C). The reaction using the same starting material in the presence of Ag²⁺ salts has also been described (Can. J. Chem. 1969, 47 1649). More recent approach is based on an oxidation of MAG with Dess-Martin periodinane (Science 2004, 305, 1752). The reaction is performed in dichlorometane (DCM). Intermediate MAG is synthesized with a monoacetylation of ethylene glycol with trimethyl orthoacetate (MeC(OMe)₃) in presence of toluenesulfonic acid monohydrate (Scheme 2).

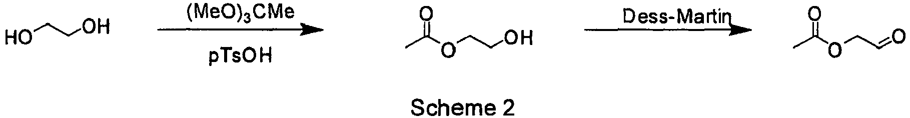

The object of the present invention is to provide an improved process for preparing statins and pharmaceutically acceptable salts thereof.

### Summary of the Invention

The present invention provides the following items including main aspects and preferred embodiments, which respectively alone and in combination particularly contribute to solving the above object and eventually provide additional advantages:
1) A process for preparing a statin compound or pharmaceutically acceptable salt thereof, comprising the steps of
   a) carrying out a process for preparing the compound of formula AOA 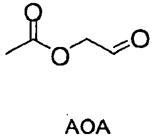 comprising the steps of:
      providing a compound of formula MAG: 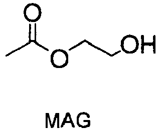 and converting the compound of formula MAG by oxidation using an N-oxyl compound in the presence of co-oxidant into the compound of formula AOA, and
   b) subjecting the compound of formula AOA to further synthesis steps to yield a statin compound or pharmaceutically acceptable salts thereof.
2) The process according to item (1), wherein said N-oxyl compound is 2,2,6,6-tetramethylpiperidine-N-oxyl.
3) The process according to item (2), wherein 2,2,6,6-tetramethylpiperidine-N-oxyl is used in catalytic amounts, preferably in molar ratio of 0.005 : 1 to 0.05 : 1 compared to compound of formula MAG
4) The process according to any one of items (1) to (3) wherein said co-oxidant is optionally used in the presence of a base, preferably said base is inorganic base, selected from the group consisting of hydroxides, carbonates and phosphates, most preferably the sodium hydrogenecarbonate is used.
5) The process according to any one of items (1) to (4), wherein said co-oxidant is a source of active halogen, preferably chlorine or bromine in oxidation state higher than -1.
6) The process according to any one of items (1) to (5) wherein said co-oxidant is selected from the group consisting of:
   - inorganic salts, preferably selected from alkali metal and earth alkali metal hypohalogenites, said hypohalogenites are preferably selected from hyypochlorites and hypobromites, said hypochlorite is most preferably calcium hypochlorite (Ca(OCl)₂) and said hypobromite is most preferably calcium hypobromite (Ca(OBr)₂),
   - active halogen containing organic compounds, preferably selected from the group consisting of N-halo substituted compounds selected from N-halo imides (said N-halo imide is preferably N-bromosuccinimide), N-halo hydantoins (said N-halo hydantoin is preferably 1,3-dibromo-5,5-dimethylhydantoin) and isocyanuric derivatives of formula A 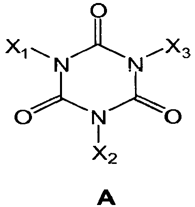 or salts thereof, wherein anyone of X₁, X₂, X₃ is selected from the group consisting of H, Cl, Br, and I, and at least one of X₁, X₂, X₃ is not H; said isocyanuric derivative is preferably selected from the group consisting of trichloroisocyanuric acid, dichloroisocyanuric acid or a salt thereof and tribromoisocyanuric acid, most preferably said isocyanuric derivative is selected from the group consisting of potassium or sodium salt of dichloroisocyanuric acid and trichloroisocyanuric acid, and
   - combinations thereof.
7) The process according to any one of items (5) to (6), further characterized in that when co-oxidant is calcium hypochlorite (Ca(OCl)₂) and / or calcium hypobromite (Ca(OBr)₂) said co-oxidant is used in molar ratio of 0.5 : 1 to 1 : 1, preferably in molar ratio of 0.5 : 1 to 0.65 : 1, compared to compound of formula MAG.
8) The process according to any one of items (5) to (7), wherein co-oxidant is a solid calcium hypochlorite in combination with inorganic or quaternary ammonium bromide, preferably said inorganic bromide is sodium bromide.
9) The process according to any one of items (5) to (6), further characterized in that when co-oxidant is trichloroisocyanuric acid said co-oxidant is used in molar ratio of 0.3 : 1 to 2 : 1, preferably in molar ratio of 0.8 : 1 to 1 : 1, compared to compound of formula MAG.
10) The process according to any one of items (1) to (9), wherein said process is performed in non-aqueous solvent, said non-aqueous solvent is preferably selected from the group consisting of saturated hydrocarbons, esters, tertiary alcohols, chlorinated solvents and mixtures thereof, more preferably non-aqueous solvent is selected from the group consisting of chlorinated hydrocarbons and mixtures of tertiary alcohols with esters, most preferably non-aqueous solvent is selected from the group consisting of dichloromethane, mixture of t-butyl alcohol with ethyl acetate and mixture of t-amyl alcohol with ethyl acetate.
11) The process according to item (10), wherein the content of water in said non-aqueous solvent is below 0.5 % by weight, more preferably below 0.1 % by weight, most preferably below 0.01 % by weight.
12) The process according to any one of items (1) to (11), wherein said compound of formula MAG 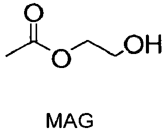 is prepared by a process comprising esterification of ethylene glycol.
13) The process according to item (12) wherein said esterification of ethylene glycol is performed in the presence of acetic acid, optionally in the presence of strong acid catalyst, preferably said strong acid catalyst is selected from the group consisting of perchloric, phosphoric and sulphuric acid.
14) The process according to item (12) wherein said esterification of ethylene glycol is performed using diacetyl ethylene glycol in the presence of basic catalyst.
15) The process according to item (14), wherein ethylene glycol is used in molar ratio of 1 : 1 to 2 : 1, preferably in molar ratio of 1.05 : 1 to 1.20 : 1, most preferably in molar ratio of 1.10 : 1 to 1.15 : 1, compared to diacetyl ethylene glycol.
16) The process according to any one of items (14) to (15), wherein said basic catalyst is selected from the group consisting of alkali metals (preferably sodium), alkali metal alcoholates and earth alkali metal alcoholates (preferably potassium tert-butylate), hydrides (preferably sodium hydride), amides (preferably lithium diisopropylamide), hydroxides (preferably sodium hydroxide) and silylamides (preferably sodium hexamethyldisilazane), most preferably sodium hydride is used.
17) The process according to any one of items (12) to (16), wherein the esterification reaction of ethylene glycol is carried out by reacting ethylene glycol and acetic acid, optionally with but preferably without further acid or base catalyst, at a temperature of higher than 100°C, preferably at a temperature of higher than 120°C.
18) The process according to any one of items (12) - (17) further comprising the steps of:
   a) diluting the reaction mixture obtained in esterification step with water,
   b) removing diacetyl ethylene glycol by extraction from the polar solvent solution from step a) with apolar solvent selected from the group of hydrocarbons, preferably toluene is used,
   c) subsequently extracting MAG from the polar solvent solution from step b) with a solvent of medium polarity selected from the group consisting of esters and chlorinated hydrocarbons, and
   d) evaporating said solvent of medium polarity.
19) The process according to item (18) further characterized in that when the esterification step is performed using diacetyl ethylene glycol in the presence of basic catalyst, step a) is optionally followed by neutralisation using appropriate acid, preferably solution of phosphoric acid (85 %) is used.
20) The process according to any one of items (1) to (19), further comprising, after the compound of formula AOA is prepared, purifying AOA by filtration and/or isolating AOA by distillation.
21) A process for the preparation of a statin compound or pharmaceutically acceptable salt thereof, comprising the steps of preparing the compound of formula AOA 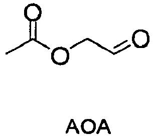 comprising the steps of:
   a) monoacetylating of ethylene glycol by acetic acid optionally in the presence of catalytic amounts of non-volatile strong acids
   b) diluting the reaction mixture from step a) with water
   c) removing diacetyl ethylene glycol by extraction from the polar solvent solution from step b) with apolar solvent selected from the group of hydrocarbons, preferably toluene is used
   d) subsequently extracting compound of formula MAG from the polar solvent solution from step c) with a solvent of medium polarity selected from the group consisting of esters and chlorinated hydrocarbons
   e) evaporating said solvent of medium polarity
   f) oxidizing compound of formula MAG obtained in step e) 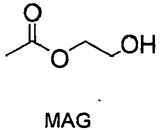 with N-oxyls, preferably TEMPO in the presence of active halogen compounds in non-aqueous media
   g) filtering off insoluble parts of the reaction suspension
   h) evaporating solvents to yield crude AOA and,
   i) optionally distilling AOA
   j) subjecting the compound of formula AOA to further synthesis steps to yield a statin compound or pharmaceutically acceptable salts thereof.
22) A process for the preparation of a statin compound or pharmaceutically acceptable salt thereof, comprising the steps of preparing the compound of formula AOA 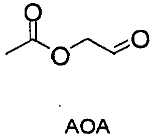 comprising the steps of:
   a) esterification of ethylene glycol using diacetyl ethylene glycol in the presence of basic catalyst
   b) diluting the reaction mixture obtained in esterification step with water optionally followed by neutralisation using appropriate acid, preferably solution of phosphoric acid (85 %) is used
   c) removing diacetyl ethylene glycol by extraction from the polar solvent solution from step b) with apolar solvent selected from the group of hydrocarbons, preferably toluene is used
   d) subsequently extracting compound of formula MAG from the polar solvent solution from step c) with a solvent of medium polarity selected from the group consisting of esters and chlorinated hydrocarbons
   e) evaporating said solvent of medium polarity
   f) distilling evaporation residue to obtain MAG, preferably of purity above 95 % (GC) and with low content of water
   g) oxidizing compound of formula MAG obtained in step f) with N-oxyls, preferably TEMPO in the presence of active halogen compounds in non-aqueous media
   h) filtering off insoluble parts of the reaction suspension
   i) evaporating solvents to yield crude AOA and,
   j) optionally distilling AOA
   k) subjecting the compound of formula AOA to further synthesis steps to yield a statin compound or pharmaceutically acceptable salts thereof.
23) The process according to any one of items (21) to (22) optionally further comprising the step of evaporating of apolar phase from step c), distillation of obtained residue and obtaining DAG which can be recovered and reused.
24) The process according to any one of items (21) to (22) optionally further comprising the step of distillation of the polar phase from step d) and obtaining EG which can be recovered and reused.
25) The process according to item (1), wherein said statin compound is selected from the group consisting of rosuvastatin, cerivastatin, fluvastatin, pitavastatin and bervastatin, preferably said statin compound is rosuvastatin.
26) A process for the preparation of a pharmaceutical composition comprising rosuvastatin as active ingredient, comprising the steps of:
   a) preparing rosuvastatin or pharmaceutically acceptable salts thereof according to the process according to item (1), and
   b) admixing the thus prepared rosuvastatin or pharmaceutically acceptable salt thereof with at least one pharmaceutically acceptable excipient.

According to the present invention, it has been surprisingly found that a more efficient, easier to handle and industrially applicable synthesis of statin compounds or pharmaceutically acceptable salts thereof can be carried out, said synthesis comprising a new preparation of acetoxyacetaldehyde , by selecting suitable starting materials which can be converted to the desired product without the necessity of aggressive, difficult to handle and/or expensive reagents (e. g. OsO₄, NalO₄, ozone, silver salts, the Dess-Martin reagent) as well as without the necessity of using extreme conditions (e. g. very high temperatures and / or very low temperatures). Starting materials applied in the procedural concept according to the present invention are cheap and easily accessible. Moreover, the process for the preparation as described above is more efficient as it allows beneficial reaction conditions due to the use of non-aqueous solvents (as a polyfunctional molecule acetoxyacetaldehyde is prone to polymerisation, hydrolysis, acetalation or hydration especially in the presence of water) providing for less by-products and thus higher purity of the products and higher yields, and/or less necessary reaction steps. The process as described above can further be advantageously carried out in various solvent systems or solvents and is thereby not limited to one particular solvent system or solvent, unless the reaction is significantly affected while being performed in certain particular solvent systems or solvents.

In addition it has surprisingly been found that a catalytic oxidation using an N-oxyl compound in the presence of co-oxidant can be made highly efficient when starting with monoacetylated monoethylene glycol, in particular when such monoacetylated monoethylene glycol can be provided in pure form. In a preferred embodiment, provision of monoacetylated monoethylene glycol in pure form as an intermediate of a 2 reaction step process can be ensured by monoacetylation of the starting diol using esterification or transesterification. Furthermore a process according to present invention as defined above is suitable for continuous production of acetoxyacetaldehyde with re-usage of solvents as well as by-products in subsequent batches resulting in minimum amounts of waste material. Such continuous process is superior from ecological point of view and appropriate for large scale industrial production.

As a result, desirable building block which is particularly suited for the preparation of statins or pharmaceutically acceptable salts thereof is provided by a significantly improved process.

### Detailed Description of Invention

In the following, the present invention will be described in more detail by preferred embodiments and examples noting, however, that these embodiments, examples are presented for illustrative purposes only and shall not limit the invention in any way.

The term "about" generally means within 10%, preferably 5% and more preferably within 1 % of a given value or range. Alternatively, the term "about" means within an acceptable standard error of the mean, when considered by one of the ordinary skill in the art.

An advantage of the present invention is the provision of a cost competitive, industrially applicable process involving the obtainment of acetoxyacetaldehyde (AOA) from derivatives of ethylene glycol. A whole procedure from ethylene glycol via MAG to yield AOA involves only two chemical steps. With simple purification methods such as a distillation a material with at least 75 % assay of AOA and less than 10 % of low molecular impurities can be provided. The procedure allows to employ cheap raw materials like ethylene glycol, its acetylated derivatives, acetic acid, strong inorganic acids, simple active halogen containing compounds, and 2,2,6,6-tetramethylpiperidine-N-oxyl (TEMPO). TEMPO as the most expensive raw material in this process can be used only in catalytic amounts. Acetoxyacetaldehyde is prepared by oxidation of monoacetyl ethylene glycol (MAG) with a co-oxidant (preferably active halogen, more preferably chlorine) in the presence of N-oxyls, preferably TEMPO, wherein catalytic amounts of an N-oxyl compound are sufficient. Said method using sodium hypochlorite in water-containing media is well known for oxidation of O-substituted glycols but if applied for preparation of AOA massive polymerisation and degradation of AOA would occur.

A surprising finding of the present inventors however is that AOA can be isolated in high yield and purity if the N-oxyl/oxidant mediated oxidation is carefully performed in suitable media, in particular if the solvent medium is non-aqueous having, at most, only limited amount of water and preferably is free or essentially free of water or contains water only at a natural level. Subsequently, isolation of AOA can in the following be accomplished by careful distillation. The final product is stable at temperatures below room temperature (preferably at temperatures below 10°C) and any possible polymerisation is, if present at all, very slow. Furthermore it is possible to redistill the stored compound before use to depolymerise it and to reduce impurities.

According to particularly preferred embodiment, AOA according to present invention is prepared from MAG via oxidation by N-oxyls, preferably TEMPO, with active halogen compounds as co-oxidants in non-aqueous media. The preferred approach according to present invention is shown in Scheme 3 in which /X/ designates a co-oxidant that is a source of active halogen, wherein said active halogen is a halogen, preferably chlorine or bromine in oxidation state higher than -1 and is preferably but not necessarily "hypohalogenite" (*i.e*. having formal oxidation state +1).

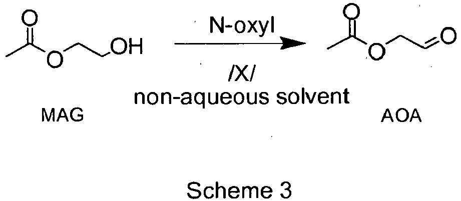

According to one aspect of invention the co-oxidant is selected from inorganic salts, preferably alkali metal and earth alkali metal hypohalogenites, preferably hypochlorites and hypobromites. Most preferably calcium hypochlorite (Ca(OCl)₂) or calcium hypobromite (Ca(OBr)₂) are used in molar ratio of 0.5 : 1 to 1 : 1 (hypohalogenite ratio of 1 : 1 to 2 : 1), preferably in molar ratio of 0.5 : 1 to 0.65 : 1 (hypohalogenite ratio of 1 : 1 to 1.3 : 1), TEMPO is preferably used in catalytic amounts, most preferably in molar ratio of 0.005 : 1 to 0.05 : 1 compared to compound of formula MAG.

The reaction is preferably performed in non-aqueous solvent. Non-aqueous solvent according to present invention should be a solvent or a mixture of solvents which is not susceptible to oxidation under reaction conditions applicable according to present invention and to which no additional amount of water is added. Preferably any content of water, if present at all, is below 0.5%, more preferably bellow 0.1%, most preferably bellow 0.01% by weight. In a usual case a solvent generally includes a natural amount of water carried to the reaction vessel from commercial solvents, reagents and MAG from previous step, but in certain cases or if desired it is preferable to dry solvents additionally using conventional drying agents not limitingly selected from the group consisting of anhydrous sulphates, calcium chloride, calcium hydride, and/or using molecular sieves or removing water with azeotropic distillation (e.g. using dichloromethane)

Solvents are preferably selected from the group consisting of saturated hydrocarbons, esters, tertiary alcohols, chlorinated solvents and mixtures thereof. Most preferably chlorinated hydrocarbon (such as dichloromethane) or a mixture of a tertiary alcohol and an ester (such as a combination of t-butyl alcohol with ethyl acetate or t-amyl alcohol with ethyl acetate) is used. Reaction is performed for a period of 0.5 to 24 hours, preferably for a period of 1 to 15 hours, more preferably for a period of 7 to 10 hours when inorganic hypochlorite is used or for a period of 1 to 3 hours when organic chloro compound is used. The oxidation is preferably performed at temperatures between -30 °C and 60 °C more preferably at temperatures between 0°C to 40°C, most preferably at about room temperature.
After reaction completion insoluble parts of suspension are filtered off and the solvent is evaporated to yield crude AOA as a yellow liquid. AOA is then purified, preferably using distillation.

As another aspect, the present invention involves an improved synthesis of the starting monoacetyl ethylene glycol (MAG). MAG is a very simple molecule which can be prepared from widespread and cheap ethylene glycol (EG) but acetylation with simple acetic derivatives is limited by considerable level of side reaction of overacetylation. Many approaches to favour monoacetylation are known in literature such as use of equimolar amounts of more reactive acetylation agents like acetanhydride, triethyl ortoacetate, acetic esters or use of additives like FeCl₃ (Jiangsu Huagong 2003, **31**, 33), zeolites (Synlett **2003**, 2419). A special isolation

TEMPO is preferably used in catalytic amounts, most preferably in molar ratio of 0.005 : 1 to 0.05 : 1 compared to compound of formula MAG.

The reaction is preferably performed in non-aqueous solvent. Non-aqueous solvent according to present invention should be a solvent or a mixture of solvents which is not susceptible to oxidation under reaction conditions applicable according to present invention and to which no additional amount of water is added. Preferably any content of water, if present at all, is below 0.5%, more preferably bellow 0.1 %, most preferably bellow 0.01% by weight. In a usual case a solvent generally includes a natural amount of water carried to the reaction vessel from commercial solvents, reagents and MAG from previous step, but in certain cases or if desired it is preferable to dry solvents additionally using conventional drying agents not limitingly selected from the group consisting of anhydrous sulphates, calcium chloride, calcium hydride, and/or using molecular sieves or removing water with azeotropic distillation (e.g. using dichloromethane)

Solvents are preferably selected from the group consisting of saturated hydrocarbons, esters, tertiary alcohols, chlorinated solvents and mixtures thereof. Most preferably chlorinated hydrocarbon (such as dichloromethane) or a mixture of a tertiary alcohol and an ester (such as a combination of t-butyl alcohol with ethyl acetate or t-amyl alcohol with ethyl acetate) is used. Reaction is performed for a period of 0.5 to 24 hours, preferably for a period of 1 to 15 hours, more preferably for a period of 7 to 10 hours when inorganic hypochlorite is used or for a period of 1 to 3 hours when organic chloro compound is used. The oxidation is preferably performed at temperatures between -30 °C and 60 °C more preferably at temperatures between 0°C to 40°C, most preferably at about room temperature.
After reaction completion insoluble parts of suspension are filtered off and the solvent is evaporated to yield crude AOA as a yellow liquid. AOA is then purified, preferably using distillation.

Further aspect of present invention is an improved synthesis of the starting monoacetyl ethylene glycol (MAG). MAG is a very simple molecule which can be prepared from widespread and cheap ethylene glycol (EG) but acetylation with simple acetic derivatives is limited by considerable level of side reaction of overacetylation. Many approaches to favour monoacetylation are known in literature such as use of equimolar amounts of more reactive acetylation agents like acetanhydride, triethyl ortoacetate, acetic esters or use of additives like FeCl₃ (Jiangsu Huagong 2003, **31**, 33), zeolites (Synlett **2003**, 2419). A special isolation approach according to present invention using a double set of solvents surprisingly separates the overacetylated by-product efficiently while a conversion of the diacetyl product to the wanted monoacetyl glycol enables a regeneration of overacetylated product with minor losses.

Ethylene glycol may be converted to MAG by esterification with acetic acid optionally in the presence of strong acid catalyst (Scheme 4).

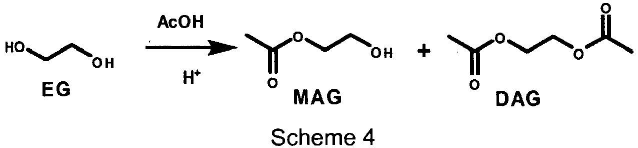

Esterification and transesterification in acidic conditions is an equilibrium process and such approach leads to formation of diacetyl glycol (DAG) and remaining considerable amounts of unreacted ethylene glycol.

According to present invention in order to limit diacetylation, excess of ethylene glycol compared to acetic acid is preferably used. Thus ethylene glycol is heated suitably with 0.1 to 10, preferably 0.3 to 0.5 molar equivalents of acetic acid in the presence of a non-volatile strong inorganic acid, preferably perchloric, phosphoric or sulphuric acid or in the presence of acidic solid supporters (*e.g*. Amberlist^{®} 15). The aforementioned process is preferably exercised at the temperatures between 0 °C to 150 °C, more preferably at temperatures between 0 °C to 50 °C, most preferably at around room temperature for a period of 1 to 24 hours. The main product of such reaction is MAG but considerable range of diacetylation also occurs.

One way to limit diacetylation may be a continuous removal of formed MAG from equilibrium by continuous extraction with organic solvents Furthermore, ethylene glycol and acetic acid may be continuously added and converted to MAG. Such trials were not very successful. When apolar solvents such as hydrocarbons were used predominately less polar diacetylated product is extracted. If however solvents with properties of Lewis base, such as ethers were used, acetic acid is predominately extracted and reaction is stopped not allowing considerable acetylation to take place.

DAG is less polar than MAG so its extraction is faster than MAG. It is therefore cumulated in organic solvent in spite of minor formation in the reaction mixture.
Acetic acid can also be extracted by organic solvent which may stop the reaction and on the other hand may catalytically re-equilibrate the ratio of glycol components in organic extract to detriment of MAG.

Further approach of limiting diacetylation is to lower the speed of reaction by reducing amount of strong acid. A slower reaction could reduce diacetylation. It has been surprisingly found that the esterification occurs even in the complete absence of strong acid if the reaction is carried out at high temperature. Acetic acid, besides being a reagent, may due to its weak acidity also have a catalytic role. Satisfactory equilibrium ratio DAG : MAG of 1:3 is reached that may form a sound base for a competitive and cheap production of MAG if an efficient separation of DAG from MAG and its further recovery is performed.
In a preferred embodiment of the present invention, a mixture of ethylene glycol and acetic acid in molar excess compared to ethylene glycol (ethylene glycol is preferably used in molar ratio of 1 : 1 to 2 : 1, more preferably in molar ratio of 1.05 : 1 to 1.20 : 1, most preferably in molar ratio of 1.10 : 1 to 1.15 : 1, compared to diacetyl ethylene glycol.) is heated to temperatures above 100 °C, preferably to temperatures above 120 °C, most preferably to temperature of about 130 °C for a period of 1 - 10 hours, preferably for a period of 2 - 4 hours. The obtained glycol ratio EG : MAG : DAG after the reaction completition shows at least twice weight amount of MAG in comparison to EG or DAG preferably the weight ratio is 15-25 : 50-70 : 15-25.
Ethylene glycol can be transformed to MAG by the process of transesterification in acidic conditions using cheap ester, preferably ethyl acetate. It is an equilibrium reaction again and all three analogues of glycol are isolated. Better approach is by using basic catalysis because the reaction is mainly directed to acetylation products. However when ethyl acetate as a transesterification reagent is used, considerable amounts of diacetylation still cannot be avoided.

The inventors of present invention have surprisingly found that if transesterification of ethylene glycol (EG) is performed with diacetyl ethylene glycol (DAG) in basic conditions most of DAG is consumed and MAG is formed in a great excess (Scheme 5). DAG plays a role of acetylating agent and theoretically one mole of ethylene glycol and one mole of DAG will give two moles of MAG. Ethylene glycol is preferably used in amount of 1 to 2 molar equivalents, more preferably in amount of 1.05 to 1.2 molar equivalents, most preferably in amount of 1.1 to 1.15 molar equivalents compared to diacetyl ethylene glycol. Preferably an excess of cheaper ethylene glycol is used to consume as much of DAG as possible. All compounds which form ethylene glycol anion can be applied in the reaction as basic catalysts, preferably said basic catalyst is selected from the group consisting of alkali metals (preferably sodium), alkali metal and earth alkali metal alcoholates (preferably potassium tert-butylate), hydrides (preferably sodium hydride), amides (preferably lithium diisopropylamide), hydroxides (preferably sodium hydroxide) and silylamides (preferably sodium hexamethyldisilazane), most preferably sodium hydride is used. The reaction is preferably carried out at temperatures between 0 and 100 °C for a period of 0.5 to 10 hours, more preferably at about room temperature for a period of 1 to 2 hours.

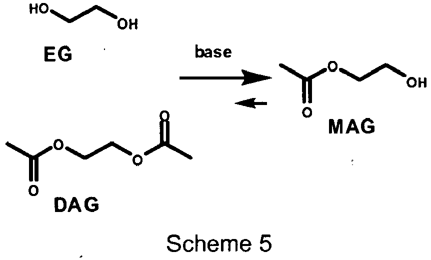

The reaction mixture is further optionally neutralised by using strong involatile inorganic acids such as phosphoric acid followed by dilution with water.

According to present invention the mixture of glycols prepared according to aforementioned procedures using acid catalysed acetylation, acetylation with acetic acid alone or basic catalysed transesterification is further treated according to the following extraction procedure. The reaction mixture which is previously diluted with water and optionally neutralised is first extracted with apolar solvent or a mixture of apolar solvents, selected from hydrocarbons, most preferably toluene, to eliminate unreacted DAG. Afterwards the polar phase is extracted with a solvent of middle polarity or a mixture of such solvents, selected from the group consisting of esters, water immiscible ketones and chlorinated hydrocarbons, preferably dichloromethane is used. Solvent of medium polarity is removed by evaporation to obtain MAG with low water content. The apolar solvent is removed by evaporation to give mainly DAG which can be reused in the next batch. Polar residue can be distilled to give mainly EG which can also be reused in the next batch. Double extraction approach with two sets of water immiscible solvents with different polarity is more efficient when higher excess of MAG over DAG is achieved in esterification step. Said approach is therefore more suitable for basic catalysed transesterification but can also be used in various acid catalysed acetylations. Additionally most of unwanted DAG can be reused in subsequent batch resulting in most of DAG being converted to MAG. According to said procedure MAG of chromatographic purity of more than 95 % is preferably prepared.

Evaporation residue from the extract with solvents of medium polarity, which predominantly contains MAG, is optionally purified by distillation.

Evaporation residue from the extract with apolar solvents, which predominantly contains DAG, is optionally purified by distillation. In the case that DAG is reused there is no need for further purification and the presence of additional amounts of MAG and EG is acceptable.

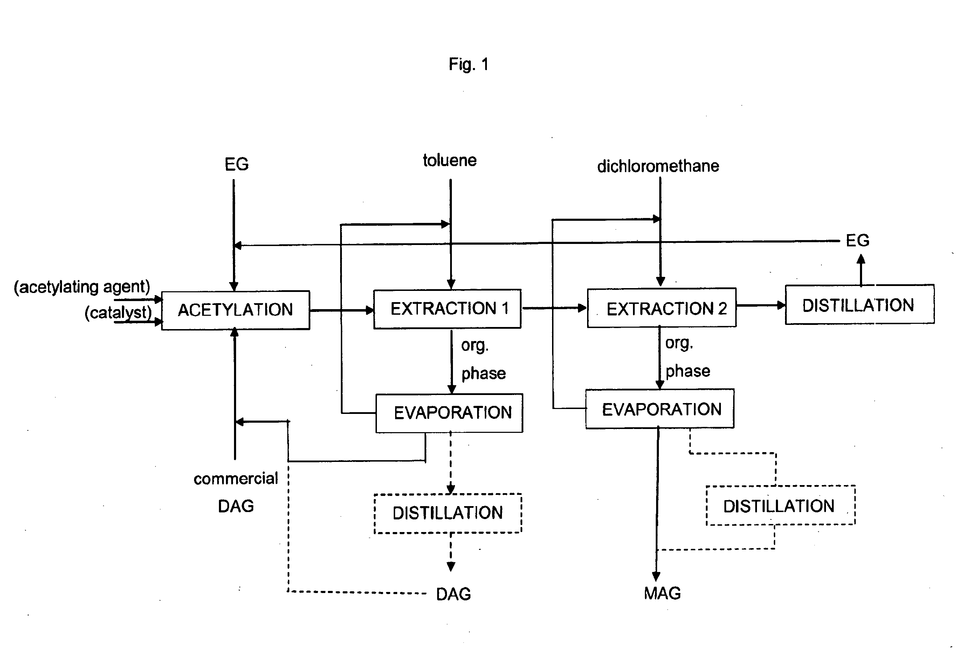

In summary the recovered solvents can be used in the next batch and the whole process can be performed continuously. Ethylene glycol is collected, optionally redistilled and used in another batch. DAG is collected and can be converted to MAG in the next batch. Fig. 1 schematically represents this procedure and shows that most of material is consumed and only minor amounts of residues after distillation are eliminated as waste. Such continuous process is superior from ecological point of view and appropriate for large scale industrial production.
d) subsequently extracting MAG from the polar solvent solution from step c) with a solvent of medium polarity selected from the group consisting of esters and chlorinated hydrocarbons
e) optionally evaporating said solvent of medium polarity
f) distilling evaporation residue from step e) to obtain MAG of purity above 95 % (GC) and of low content of water
g) oxidizing MAG with N-oxyls, preferably TEMPO in the presence of active halogen compounds in non-aqueous media
h) filtering off insoluble parts of the reaction suspension
i) evaporating solvents to yield crude AOA and,
j) optionally distilling AOA to give colourless or slightly yellowish liquid which predominantly contains monomer.

Optionally the apolar phase of step c) is evaporated, residue distilled obtaining DAG which can be recovered and reused.

Optionally the polar phase of steps d) is distilled obtaining EG which can be recovered and reused.

The acetoxyacetaldehyde is then subjected to further synthesis steps, in order to yield statin compounds or pharmaceutically acceptable salts thereof by synthesis routes known to or readily devisable by a person skilled in the art. For example, various acetaldehydes can be converted by DERA enzymes to lactols (Scheme 6) which are 6 carbon atom synthons for side chain of various HMG CoA reductase inhibitors ("statin compounds", or simply "statins"). 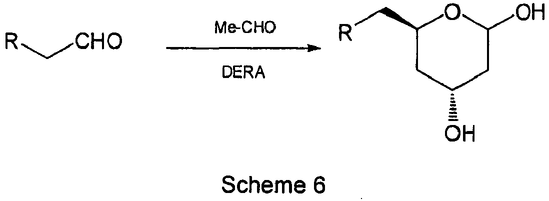 Lactol (R = OAc) which can be prepared from AOA can be converted to different synthons for synthesis of statins (Scheme 7).
d) subsequently extracting MAG from the polar solvent solution from step c) with a solvent of medium polarity selected from the group consisting of esters and chlorinated hydrocarbons
e) optionally evaporating said solvent of medium polarity
f) distilling evaporation residue from step e) to obtain MAG of purity above 95 % (GC) and of low content of water
g) oxidizing MAG with N-oxyls, preferably TEMPO in the presence of active halogen compounds in non-aqueous media
h) filtering off insoluble parts of the reaction suspension
i) evaporating solvents to yield crude AOA and,
j) optionally distilling AOA to give colourless or slightly yellowish liquid which predominantly contains monomer.

Optionally the apolar phase of step c) is evaporated, residue distilled obtaining DAG which can be recovered and reused.

Optionally the polar phase of steps d) is distilled obtaining EG which can be recovered and reused.

The acetoxyacetaldehyde then can be a useful raw material and can be subjected to further synthesis steps, preferably in order to yield different active pharmaceutical ingredients, preferably statins, by synthesis routes known to or readily devisable by a person skilled in the art. For example, various acetaldehydes can be converted by DERA enzymes to lactols (Scheme 6) which are 6 carbon atom synthons for side chain of various HMG CoA reductase inhibitors ("statin compounds", or simply "statins").

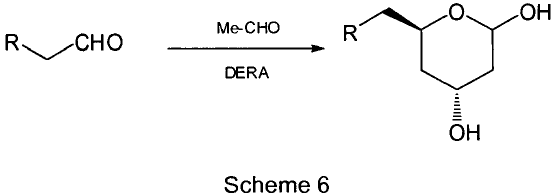

Lactol (R = OAc) which can be prepared from AOA can be converted to different synthons for synthesis of statins (Scheme 7).

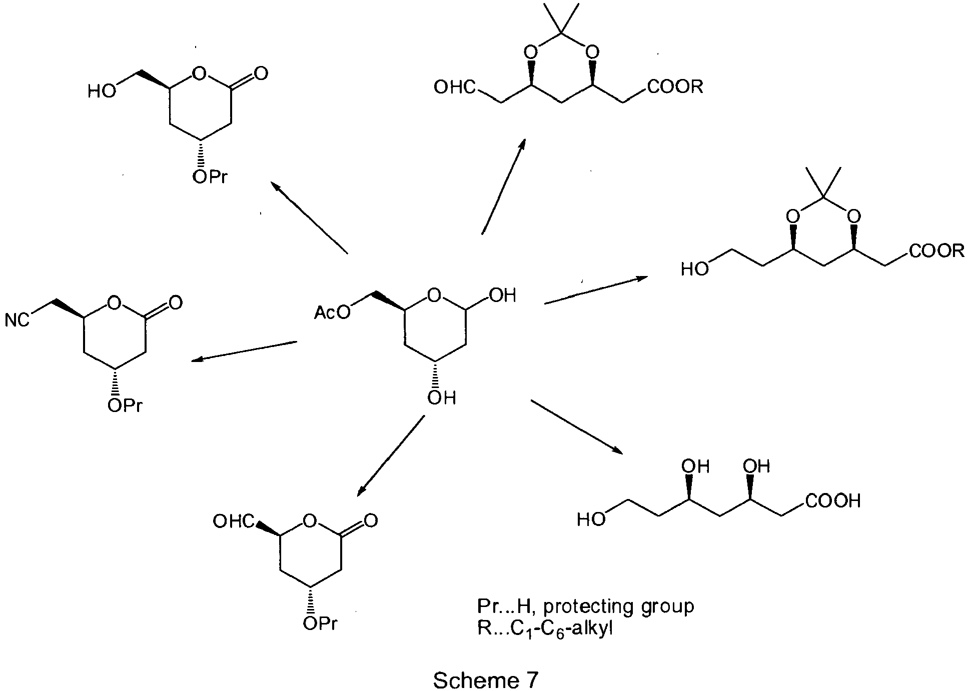

In a representing but not limiting example the lactol A is oxidized to the lactone B, protected with a sylyl group to the lactone C, further hydrolyzed to the hydroxy derivative D and oxidized to the aldehyde E which is suitable formation for Wittig-Horner coupling with various phosphorous compounds Stat-CH₂-W in which Stat represents the main part of a statin or a structure which could be converted to the main part of the statin and W represents phosphonium, phosphinoxide or phosphonic ester (Scheme 8).

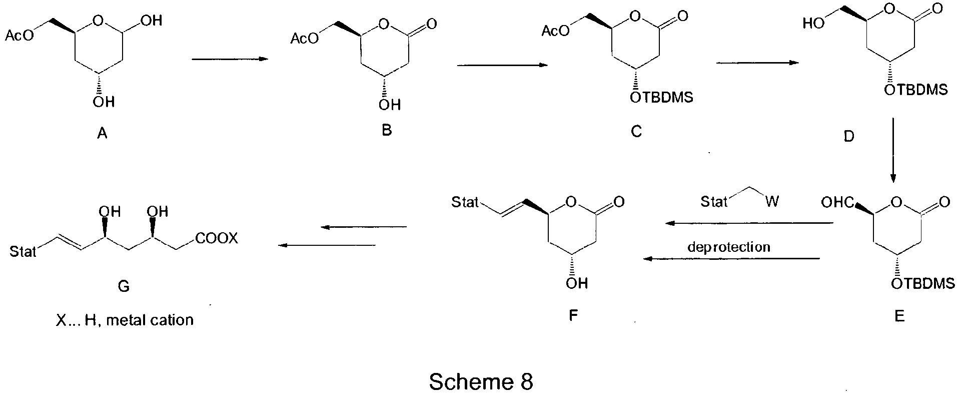

Statins which can be prepared by such approach are preferably selected from the group consisting of rosuvastatin, cerivastatin, fluvastatin, pitavastatin and bervastatin, isolated in a lactone form of Formula F or in an open chain form of formula G in which Stat represents structures shown in Scheme 9, and in which X is preferably alkali metal or earth alkali metal cation. In the most preferred case rosuvastatin (Formula G, Stat = Stat1, X=Ca_{1/2}) is prepared.

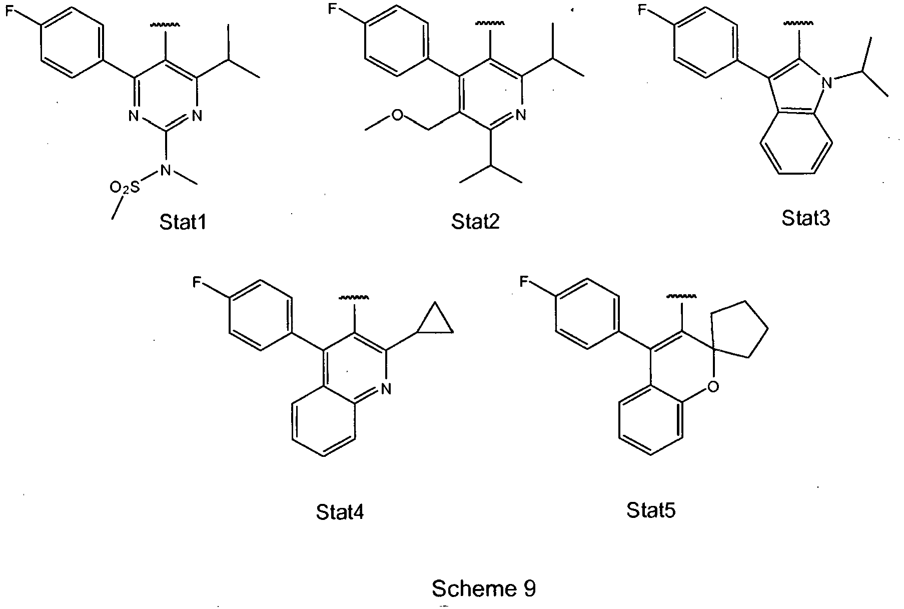

For preparing a pharmaceutical composition comprising abovementioned statins, preferably rosuvastatin or pharmaceutically acceptable salts thereof as active ingredient, first statin, preferably rosuvastatin or pharmaceutically acceptable salts thereof is provided by the process as described above.

Then, the thus prepared statin, preferably rosuvastatin or pharmaceutically acceptable salts thereof is suitably admixed with at least one suitable pharmaceutically acceptable excipient. Pharmaceutically acceptable excipients may be selected from the group consisting of binders, diluents, disintegrating agents, stabilizing agents, preservatives, lubricants, fragrances, flavoring agents, sweeteners and other excipients known in the field of the pharmaceutical technology.
Preferably, excipients may be selected from the group consisting of lactose, microcrystalline cellulose, cellulose derivatives, e.g. hydroxypropylcellulose, polyacrylates, calcium carbonate, starch, colloidal silicone dioxide, sodium starch glycolate, talc, magnesium stearate, polyvinylpyrrolidone, polyethylene glycol and other excipients known in the field of the pharmaceutical technology.

### Experimental Procedures

### Reference example A:

Ethylene glycol (EG) (62.1 mmol), acetic acid (46.57 mL) and H₂SO₄ (0.29 mL) were diluted with water (93.14 mL) and carried to a reaction in a continuous extraction apparatus using benzene as an extracting solvent. The reaction was carried out for 5 days. The organic phase was collected and analyzed by TLC, which showed only DAG as main product.

### Reference example B:

Water phase consisting of EG (0.91 L), acetic acid (8.30 L) and 96 % H₂SO₄ (0.015 L) in water (16.5 L) was continuously extracted using reaction-extraction column (capacity 35 L) and tert-butyl methyl ether (60 L). Organic phase was collected and fresh tert-butyl methyl ether was used for extraction every 24 hours. After 24 hours samples where taken from water (EG : MAG : DAG = 97.7 : 1.9 : 0.0) and organic phase (EG : MAG : DAG = 88.4 : 8.2 : 0.5).

### Example 1:

### Synthesis of monoacetyl ethylene glycol (MAG)

Ethylene glycol (EG) (266 mmol), acetic acid (87.5 mmol) and concentrated sulfuric acid (1.8 mmol) were stirred for 24 hours at room temperature. To this solution an aqueous solution of saturated NaHCO₃ (15 mL) was added and left to stir overnight. Then water (10 mL) was added and extracted first with toluene (2 x 50 mL) and then with CH₂Cl₂ (5x 50 mL). Afterwards CH₂Cl₂ phase was collected and evaporated yielding 8.0 g of mixture of components (EG : MAG: DAG = 1.2 : 97.7 : 1.1). Yield of MAG: 28 % per EG, 86 % per AcOH.

### Example 2:

### Synthesis of MAG

Ethylene glycol (EG) (161 mmol), acetic acid (209 mmol) and Amberlist^{®} 15 (1.71 g) were stirred for 4 days at room temperature. Then solids were removed by filtration and to filtrate water (35 mL) was added and extracted first with toluene (2x 80 mL) and then with CH₂Cl₂ (3x 80 mL). The CH₂Cl₂ phase was collected and evaporated yielding 6.3 g of mixture of components (EG : MAG: DAG = 0.5 : 97.5 : 2 ), yield of MAG 37 % per EG.

### Example 3:

### Synthesis of MAG

Mixture of EG (8.06 mmol) and acetic acid (10.5 mmol) was heated at 130°C for 3 hours. After cooling the mixture of products EG : MAG : DAG in ratio 23 : 59 : 18 was obtained from which MAG was isolated using extraction procedure in Example 2 in 44 % yield, calculated per EG.

### Example 4:

### Synthesis of MAG

To a mixture of EG (322 mmol) and diacetyl ethylene glycol (DAG) (290 mmol), sodium hydride (3.2 mmol) was added and left at room temperature for 1.5 hours. To this solution 85% phosphoric acid (1.6 mmol) was added dropwise and left to stir for 0.5 hour. To this mixture distilled water (60 mL) was added and extracted with toluene (2 x 80 mL). Water phase was additionally extracted with CH₂Cl₂ (5 x 80mL). CH₂Cl₂ phase was collected and solvent evaporated to yield MAG as transparent liquid (17.5 g, yield 51 % regarding MEG and GC purity >96%). Toluene phase with manly DAG can be re-used in the next batch after evaporation of solvent.

### Example 5:

### Synthesis of MAG

To a mixture of EG (322 mmol) and diacetyl ethylene glycol (DAG) (290 mmol), sodium hydroxide (3.2 mmol) was added and left at room temperature for 1.5 hours. To this solution 85% phosphoric acid (1.6 mmol) was added dropwise and left to stir for 0.5 hour. To this mixture distilled water (60 mL) was added and extracted with toluene (5 x 40 mL). Water phase was additionally extracted with CH₂Cl₂ (8 x 80mL). CH₂Cl₂ phase was collected and solvent evaporated to yield MAG as transparent liquid (29.8 g, yield 49 % regarding overall yield and GC purity >98%). Toluene phase with manly DAG can be re-used in the next batch after evaporation of solvent.

### Example 6:

### Synthesis of acetoxyacetaldehyde (AOA)

Initially, MAG (100 mmol) was diluted with a mixture of solvents (0.1 M in 90/10 ethyl acetate - tert-butanol) in batch reactor. To a solution of MAG was then successively added calcium hypochlorite (Ca(ClO)₂ - 60 mmol), sodium bromide (5 mmol) and TEMPO (3 mmol). Reaction mixture was vigorously stirred (mechanical stirring) for 9 hours at room temperature. In order to ensure batch-to-batch repeatability and to achieve the highest possible MAG conversion (AOA yield), the fine powder (filter through sieve 0,125 mm) of calcium hypochlorite was used. Reaction mixture was then filtered to remove insoluble calcium hypochlorite and the solvent was evaporated under reduced pressure. Dark-yellowish liquid was obtained with "GC (gas chromatographic) purity" of more then 40 %. Crude AOA mixture was further purified by distillation under reduced pressure (80 °C (bath) and 20 to 13 mbar) to obtain pure AOA (purity up to 92% GC, 75% assay).

### Example 7:

### Synthesis of AOA

To a suspension of NaHCO₃ (269 mmol), trichloroisocyanuric acid (TCICA - 38.4 mmol) and MAG (45.2 mmol) in CH₂Cl₂ (100 mL) cooled on an ice bath TEMPO (0.48 mmol) was added at once. Reaction mixture was left to stir for 1.5 hours and then filtered through a pad of Celite®. Filtrate was concentrated under reduced pressure (rotavapor 50 mBar, T (bath) = 40 °C) and oily residue distilled (T (bath) = 80 °C, 10 mBar) to yield transparent liquid as AOA (2.96 g, purity up to 95 %).

### Example 8:

### Synthesis of acetoxyacetaldehyde (AOA)

To a suspension of NaHCO₃ (57,6 mmol), trichloroisocyanuric acid (TClCA - 7,68 mmol) and MAG (9,61 mmol) in EtOAc (48 mL) cooled on an ice bath TEMPO (0,10 mmol) was added at once. Reaction mixture was left to stir for 1.5 h and then filtered through a pad of Celite®. Filtrate was concentrated under reduced pressure (rotary evaporator 50 mBar, T (bath) = 40 °C) and solid residue formed. Sample was analysed by GC as AOA (1,05 g, purity 76 % GC area).

### Example 9:

### Synthesis of acetoxyacetaldehyde (AOA)

To a solution of MAG (1.04 g, 10 mmol) in acetonitrile (30 mL) was added sodium citrate (0.88 g, 0.3 eq) and TEMPO (47 mg, 0.03 eq). Sodium dichloroisocyanurate (SDIC - 1.98 g, 0.9 eq) was added at once. Reaction mixture was left to stir for 1 hour and then filtered through a pad of Celite® to give AOA solution with crude GC purity of 85%.

### Example 10:

### Synthesis of acetoxyacetaldehyde (AOA)

To a solution of MAG (1.04 g, 10 mmol) in acetonitrile (30 mL) was added sodium triisocyanurate (0.585 g, 0.3 eq) and TEMPO (47 mg, 0.03 eq). Sodium dichloroisocyanurate (SDIC - 1.54 g, 0.7 eq) was added at once. Reaction mixture was left to stir for 1 hour and then filtered through a pad of Celite® to give AOA solution with GC purity of 72%.

## Claims

1. A process for the preparation of a statin compound or pharmaceutically acceptable salt thereof, comprising the steps of
a) carrying out a process for preparing the compound of formula AOA 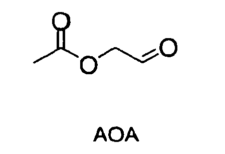 comprising the steps of:
providing a compound of formula MAG: 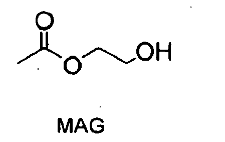 and converting the compound of formula MAG by oxidation using an N-oxyl compound in the presence of co-oxidant into the compound of formula AOA, and
b) subjecting the compound of formula AOA to further synthesis steps to yield a statin compound or pharmaceutically acceptable salts thereof.

2. The process according to claim 1, wherein said N-oxyl is 2,2,6,6-tetramethylpiperidine-N-oxyl, said 2,2,6,6-tetramethylpiperidine-N-oxyl is preferably used in catalytic amounts, more preferably in molar ratio of 0.005 : 1 to 0.05 : 1 compared to compound of formula MAG.

3. The process according to claim 1 or claim 2, wherein said co-oxidant is a source of active halogen, preferably chlorine or bromine in oxidation state higher than -1.

4. The process according to any one of claims 1 to 3 wherein said co-oxidant is selected from the group consisting of:
- inorganic salts, preferably selected from alkali metal and earth alkali metal hypohalogenites, and,
- active halogen containing organic compounds, preferably selected from the group consisting of N-halo substituted compounds selected from N-halo imides, preferaqbly N-bromosuccinimide, N-halo hydantoins, preferably 1,3-dibromo-5,5-dimethylhydantoin, and isocyanuric derivatives of formula A 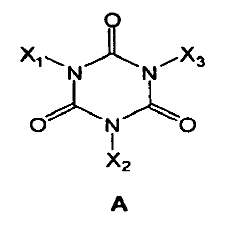 or salts thereof, wherein anyone of X₁, X₂, X₃ is selected from the group consisting of H, Cl, Br, and I, and at least one of X_{1,} X₂, X₃ is not H; said isocyanuric derivative is preferably selected from the group consisting of trichloroisocyanuric acid, dichloroisocyanuric acid and salts thereof, and tribromoisocyanuric acid, most preferably said isocyanuric derivative is trichloroisocyanuric acid or sodium salt of dichloroisocyanuric acid, and combinations thereof.

5. The process according to claim 4 wherein said hypohalogenites are selected from hypochlorites and hypobromites, preferably calcium hypochlorite (Ca(OCl₂) and calcium hypobromite (Ca(OBr)₂).

6. The process according to any one of claims 3 to 4, further **characterized in that** when co-oxidant is calcium hypochlorite (Ca(OCl)₂) and / or calcium hypobromite (Ca(OBr)₂) said co-oxidant is used in molar ratio of 0.5 : 1 to 1 : 1, preferably in molar ratio of 0.5 : 1 to 0.65 : 1, compared to compound of formula MAG.

7. The process according to claim 6 wherein said co-oxidant is used in combination with inorganic bromide, preferably sodium bromide, or quaternary ammonium bromide.

8. The process according to any one of claims 3 to 4, further **characterized in that** when co-oxidant is trichloroisocyanuric acid said co-oxidant is used in molar ratio of 0.3 : 1 to 2 : 1, preferably in molar ratio of 0.8 : 1 to 1 : 1 molar equivalents, compared to compound of formula MAG.

9. The process according to any one of claims 1 to 8, wherein said process is performed in non-aqueous solvent, preferably selected from the group consisting of saturated hydrocarbons, esters, tertiary alcohols, chlorinated solvents and mixtures thereof, from the group consisting of chlorinated hydrocarbons and mixtures of tertiary alcohols with esters, or from the group consisting of dichloromethane, mixture of t-butyl alcohol with ethyl acetate and mixture of t-amyl alcohol with ethyl acetate.

10. The process according to any one of claims 1 to 9, wherein said compound of formula MAG 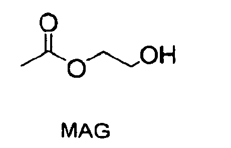 is prepared by a process comprising esterification of ethylene glycol, preferably performed in the presence of acetic acid, optionally in the presence of strong acid catalyst, preferably said strong acid catalyst is selected from the group consisting of perchloric, phosphoric and sulphuric acid.

11. The process according to claim 10 wherein said esterification of ethylene glycol is performed using diacetyl ethylene glycol in the presence of basic catalyst, wherein ethylene glycol is used in molar ratio of 1 : 1 to 2 : 1, preferably in molar ratio of 1.05 : 1 to 1.20 : 1, most preferably in molar ratio of 1.10 : 1 to 1.15 : 1, compared to diacetyl) ethylene glycol.

12. The process according to claim 10, wherein the esterification reaction of ethylene glycol is carried out by reacting ethylene glycol and acetic acid, optionally with but preferably without further acid or base catalyst, at a temperature of higher than 100°C, preferably at a temperature of higher than 120°C.

13. The process according to any one of claims 10 to 12 further comprising the steps of:
a) diluting the reaction mixture obtained in esterification step with water,
b) removing diacetyl ethylene glycol by extraction from the polar solvent solution from step a) with apolar solvent selected from the group of hydrocarbons, preferably toluene is used,
c) subsequently extracting compound of formula MAG from the polar solvent solution from step b) with a solvent of medium polarity selected from the group consisting of esters and chlorinated hydrocarbons, and
d) evaporating said solvent of medium polarity, and optionally
e) purifying prepared AOA by filtration and/or isolating AOA by distillation.

14. The process according to claim 1, wherein said statin compound is selected from the group consisting of rosuvastatin, cerivastatin, fluvastatin, pitavastatin and bervastatin, preferably said statin compound is rosuvastatin.

15. A process for the preparation of a pharmaceutical composition comprising rosuvastatin as active ingredient, comprising the steps of:
a) preparing rosuvastatin or pharmaceutically acceptable salts thereof according to the process according to claim 1, and
b) admixing the thus prepared rosuvastatin or pharmaceutically acceptable salt thereof with at least one pharmaceutically acceptable excipient.

## Patentansprüche

1. Verfahren zur Herstellung einer Statinverbindung oder einem pharmazeutisch annehmbaren Salz davon, umfassend die Schritte
a) Durchführen eines Verfahrens zur Herstellung der Verbindung der Formel AOA 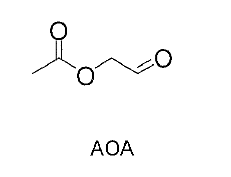 umfassend die Schritte:
Bereitstellen einer Verbindung der Formel MAG: 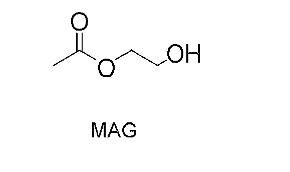 und Umwandeln der Verbindung der Formel MAG durch Oxidation unter Verwendung einer N-Oxylverbindung in der Gegenwart eines Co-Oxidans in eine Verbindung der Formel AOA, und
b) Unterwerfen der Verbindung der Formel AOA weiteren Syntheseschritten zum Erhalten einer Statinverbindung oder eines pharmazeutisch annehmbaren Salzes davon.

2. Verfahren gemäß Anspruch 1, wobei das N-Oxyl 2,2,6,6-Tetramethylpiperidin-N-Oxyl ist, wobei das 2,2,6,6-Tetramethylpiperidin-N-Oxyl vorzugsweise in katalytischen Mengen verwendet wird, insbesondere in einem molaren Verhältnis von 0,005:1 bis 0,05:1 im Vergleich zu einer Verbindung der Formel MAG.

3. Verfahren gemäß Anspruch 1 oder Anspruch 2, wobei das Co-Oxidans eine Quelle von aktivem Halogen ist, vorzugsweise Chlor oder Brom in einer Oxidationsstufe höher als -1.

4. Verfahren gemäß einem der Ansprüche 1 bis 3, wobei das Co-Oxidans ausgewählt ist aus der Gruppe bestehend aus:
- anorganischen Salzen, vorzugsweise ausgewählt aus Alkalimetall- und Erdalkalimetall-Hypohalogeniten, und
- aktives Halogen-enthaltende organische Verbindungen, vorzugsweise ausgewählt aus der Gruppe bestehend aus N-Halogen-substituierten Verbindungen ausgewählt aus N-Halogen-Imiden, vorzugsweise N-Bromsuccinimid, N-Halogen-Hydantoinen, vorzugsweise 1,3-Dibrom-5,5-dimethylhydantoin und isocyanurische Derivate der Formel A 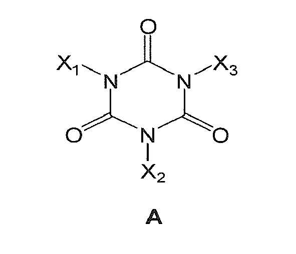 oder Salzen davon, wobei irgendeines von X₁, X₂ und X₃ ausgewählt ist aus der Gruppe bestehend aus H, Cl, Br und I und zumindest eines von X₁, X₂ und X₃ nicht H ist; wobei das isocyanurische Derivat vorzugsweise ausgewählt ist aus der Gruppe bestehend aus Trichlorisocyanursäure, Dichlorisocyanursäure und Salzen davon und Tribromisocyanursäure, insbesondere wobei das isocyanurische Derivat Trichlorisocyanursäure oder ein Natriumsalz der Dichlorisocyanursäure ist, und Kombinationen davon.

5. Verfahren gemäß Anspruch 4, wobei die Hypohalogenite ausgewählt sind aus Hypochloriten und Hypobromiten, vorzugsweise Calciumhypochlorit (Ca(OCl)₂) und Calciumhypobromit (Ca(OBr)₂).

6. Verfahren gemäß einem der Ansprüche 3 bis 4, des Weiteren **dadurch gekennzeichnet, dass**, wenn das Co-Oxidans Calciumhypochlorit (Ca(OCl)₂) und/oder Calciumhypobromit (Ca(OBr)₂) ist, das Co-Oxidans in einem molaren Verhältnis von 0,5:1 bis 1:1 verwendet wird, vorzugsweise in einem molaren Verhältnis von 0,5:1 bis 0,65:1 im Vergleich zu der Verbindung der Formel MAG.

7. Verfahren gemäß Anspruch 6, wobei das Co-Oxidans in Kombination mit anorganischem Bromid, vorzugsweise Natriumbromid oder quaternärem Ammoniumbromid, verwendet wird.

8. Verfahren gemäß einem der Ansprüche 3 bis 4, des Weiteren **dadurch gekennzeichnet, dass**, wenn das Co-Oxidans Trichlorisocyanursäure ist, das Co-Oxidans in einem molaren Verhältnis von 0,3:1 bis 2:1 verwendet wird, vorzugsweise in einem molaren Verhältnis von 0,8:1 bis 1:1 molarer Äquivalenz im Vergleich zu der Verbindung der Formel MAG.

9. Verfahren gemäß einem der Ansprüche 1 bis 8, wobei das Verfahren in einem nicht-wässrigen Lösungsmittel durchgeführt wird, vorzugsweise ausgewählt aus der Gruppe bestehend aus gesättigten Kohlenwasserstoffen, Estern, tertiären Alkoholen, chlorhaltigen Lösungsmitteln und Mischungen daraus, aus der Gruppe bestehend aus chlorierten Kohlenwasserstoffen und Mischungen aus tertiären Alkoholen mit Estern, oder aus der Gruppe bestehend aus Dichlormethan, Mischungen aus t-Butylalkohol mit Ethylacetat und Mischungen aus t-Amylalkohol mit Ethylacetat.

10. Verfahren gemäß einem der Ansprüche 1 bis 9, wobei die Verbindung der Formel MAG 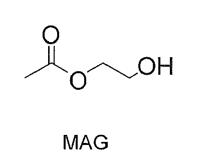 hergestellt wird durch ein Verfahren aufweisend eine Veresterung von Ethylenglykol, vorzugsweise durchgeführt in Gegenwart von Essigsäure, optional in Gegenwart eines starken sauren Katalysators, wobei der starke saure Katalysator vorzugsweise ausgewählt ist aus der Gruppe bestehend aus Perchlorsäure, Phosphorsäure und Schwefelsäure.

11. Verfahren gemäß Anspruch 10, wobei die Veresterung des Ethylenglykols unter Verwendung von Diacetylethylenglykol in Gegenwart eines basischen Katalysators durchgeführt wird, wobei das Ethylenglykol in einem molaren Verhältnis von 1:1 bis 2:1 verwendet wird, vorzugsweise in einem molaren Verhältnis von 1,05:1 bis 1,20:1, insbesondere in einem molaren Verhältnis von 1,10:1 bis 1,15:1 im Vergleich zu Diacetylethylenglykol.

12. Verfahren gemäß Anspruch 10, wobei die Veresterungsreaktion von Ethylenglykol durch Reagieren von Ethylenglykol und Essigsäure durchgeführt wird, optional mit aber vorzugsweise ohne einem weiteren sauren oder basischen Katalysator, bei einer Temperatur von höher als 100°C, vorzugsweise bei einer Temperatur von höher als 120°C.

13. Verfahren gemäß einem der Ansprüche 10 bis 12, des Weiteren aufweisend die Schritte:
a) Verdünnen der in dem Veresterungsschritt erhaltenen Reaktionsmischung mit Wasser,
b) Entfernen des Diacetylethylenglykols durch Extraktion von der polaren Lösungsmittellösung aus Schritt a) mit einem apolaren Lösungsmittel ausgewählt aus der Gruppe der Kohlenwasserstoffe, vorzugweise wird Toluol verwendet,
c) anschließendes Extrahieren der Verbindung der Formel MAG von der polaren Lösungsmittellösung aus Schritt b) mit einem Lösungsmittel einer mittleren Polarität ausgewählt aus der Gruppe bestehend aus Estern und chlorierten Kohlenwasserstoffen und
d) Verdampfen des Lösungsmittels der mittleren Polarität und optional
e) Aufreinigen des hergestellten AOA durch Filtration und/oder Isolieren des AOA durch Destillation.

14. Verfahren gemäß Anspruch 1, wobei die Statinverbindung ausgewählt ist aus der Gruppe bestehend aus Rosuvastatin, Cerivastatin, Fluvastatin, Pitavastatin und Bervastatin, wobei die Statinverbindung vorzugsweise Rosuvastatin ist.

15. Verfahren zur Herstellung einer pharmazeutischen Zusammensetzung aufweisend Rosuvastatin als aktiven Bestandteil, aufweisend die Schritte:
a) Herstellen von Rosuvastatin oder einem pharmazeutisch annehmbaren Salz davon gemäß dem Verfahren nach Anspruch 1 und
b) Vermischen des so hergestellten Rosuvastatins oder des pharmazeutisch annehmbaren Salzes davon mit mindestens einem pharmazeutisch annehmbaren Trägerstoff.

## Revendications

1. Procédé de préparation d'un composé de stanine ou d'un sel pharmaceutiquement acceptable de celui-ci, comprenant les étapes de :
a) réalisation d'un procédé de préparation du composé de formule AOA 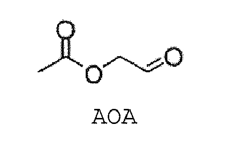 comprenant les étapes de :
fourniture d'un composé de formule MAG : 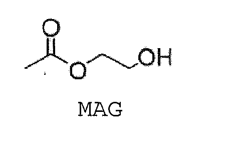 et conversion du composé de formule MAG par oxydation à l'aide d'un composé N-oxyle en présence d'un co-oxydant en composé de formule AOA, et
b) soumission du composé de formule AOA à d'autres étapes de synthèse pour produire un composé de stanine ou des sels pharmaceutiquement acceptables de celui-ci.

2. Procédé selon la revendication 1, dans lequel ledit N-oxyle est le 2,2,6,6-tétraméthylpipéridine-N-oxyle, ledit 2,2,6,6-tétraméthylpipéridine-N-oxyle est de préférence utilisé en des quantités catalytiques, mieux encore dans un rapport molaire de 0,005 : 1 à 0,05 : 1 par rapport au composé de formule MAG.

3. Procédé selon la revendication 1 ou la revendication 2, dans lequel ledit co-oxydant est une source d'halogène actif, de préférence de chlore ou de brome dans un état d'oxydation supérieur à -1.

4. Procédé selon l'une quelconque des revendications 1 à 3, dans lequel ledit co-oxydant est choisi dans le groupe constitué de :
- sels inorganiques, choisis de préférence parmi les hypohalogénites de métaux alcalins et de métaux alcalino-terreux, et
- composés organiques contenant un halogène actif, choisis de préférence dans le groupe constitué des composés N-halogéno-substitués choisis parmi les N-halogénoimides, de préférence le N-bromosuccinimide, les H-halogénohydantoïnes, de préférence la 1,3-dibromo-5,5-diméthylhydantoïne et les dérivés isocyanuriques de formule A 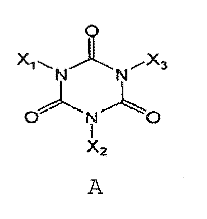 ou des sels de ceux-ci, dans laquelle l'un quelconque de X₁, X₂ et X₃ est choisi dans le groupe constitué de H, Cl, Br et I, et l'un au moins de X₁, X₂ et X₃ n'est pas H ; ledit dérivé isocyanurique est de préférence choisi dans le groupe constitué de l'acide trichloroisocyanutique, de l'acide dichloroisocyanurique et des sels de ceux-ci, et l'acide tribromoisocyanurique, tout particulièrement ledit dérivé isocyanurique est l'acide trichloroisocyanurique ou le sel de sodium de l'acide dichloroisocyanurique, et les combinaisons de ceux-ci.

5. Procédé selon la revendication 4, dans lequel lesdits hypohalogénites sont choisis parmi les hypochlorites et les hypobromites, de préférence d'hypochlorite de calcium (Ca(OCl)₂) et l'hypobromite de calcium (Ca(OBr)₂).

6. Procédé selon l'une quelconque des revendications 3 à 4, en outre **caractérisé en ce que**, lorsque le co-oxydant est l'hypochlorite de calcium (Ca(OCl)₂) et/ou l'hypobromite de calcium (Ca(OBr)₂), ledit co-oxydant est utilisé dans un rapport molaire de 0,5 : 1 à 1 : 1, de préférence dans un rapport molaire de 0,5 : 1 à 0,65 : 1, par rapport au composé de formule MAG.

7. Procédé selon la revendication 6, dans lequel ledit co-oxydant est utilisé en combinaison avec un bromure inorganique, de préférence le bromure de sodium ou le bromure d'ammonium quaternaire.

8. Procédé selon l'une quelconque des revendications 3 à 4, en outre **caractérisé en ce que**, lorsque le co-oxydant est l'acide trichloroisocyanurique, ledit co-oxydant est utilisé dans un rapport molaire de 0,3 : 1 à 2 : 1, de préférence dans un rapport molaire de 0,8 : 1 à 1 : 1 équivalent molaire, par rapport au composé de formule MAG.

9. Procédé selon l'une quelconque des revendications 1 à 8, dans lequel ledit procédé est réalisé dans un solvant non aqueux, choisi de préférence dans le groupe constitué des hydrocarbures saturés, des esters, des alcools tertiaires, des solvants chlorés et des mélanges de ceux-ci, dans le groupe constitué des hydrocarbures chlorés et des mélanges d'alcools tertiaires et d'esters, ou dans le groupe constitué du dichlorométhane, d'un mélange d'alcool t-butylique et d'acétate d'éthyle et un mélange d'alcool t-amylique et d'acétate d'éthyle.

10. Procédé selon l'une quelconque des revendications 1 à 9, dans lequel ledit composé de formule MAG 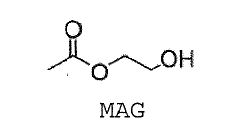 est préparé par un procédé comprenant l'estérification d'éthylène glycol, de préférence réalisé en présence d'acide acétique, éventuellement en présence d'un catalyseur de type acide fort, de préférence ledit catalyseur de type acide fort est choisi dans le groupe constitué des acides perchlorique, phosphorique et sulfurique.

11. Procédé selon la revendication 10, dans lequel ladite estérification de l'éthylène glycol est réalisée à l'aide de diacétyléthylène glycol en présence d'un catalyseur basique, dans lequel l'éthylène glycol est utilisé dans un rapport molaire de 1 : 1 à 2 : 1, de préférence dans un rapport molaire de 1,05 : 1 à 1,20 : 1, tout particulièrement dans un rapport molaire de 1,10 : 1 à 1, 15 : 1, par rapport au diacétyléthylène glycol.

12. Procédé selon la revendication 10, dans lequel la réaction d'estérification de l'éthylène glycol est réalisée par réaction d'éthylène glycol et d'acide acétique, éventuellement, mais de préférence, sans autre catalyseur acide ou basique, à une température supérieure à 100°C, de préférence à une température supérieure à 120°C.

13. Procédé selon l'une quelconque des revendications 10 à 12, comprenant en outre les étapes de :
a) dilution du mélange réactionnel obtenu dans l'étape d'estérification avec de l'eau,
b) séparation du diacétyléthylène glycol par extraction de la solution dans le solvant polaire de l'étape a), un solvant apolaire choisi dans le groupe constitué des hydrocarbures, de préférence le toluène, étant utilisé,
c) ultérieurement extraction du composé de formule MAG de la solution dans un solvant polaire de l'étape b) avec un solvant de polarité moyenne choisi dans le groupe constitué des esters et des hydrocarbures chlorés, et
d) évaporation dudit solvant de polarité moyenne, et éventuellement
e) purification de l'OAO préparé par filtration et/ou isolement de l'AOA par distillation.

14. Procédé selon la revendication 1, dans lequel ledit composé de statine est choisi dans le groupe constitué de la rosuvastatine, de la cérivastatine, de la fluvastatine, de la pitavastatine et de la bervastatine, de préférence ledit composé de statine est la rosuvastatine.

15. Procédé de préparation d'une composition pharmaceutique comprenant de la rosuvastatine en tant qu'ingrédient actif, comprenant les étapes de :
a) préparation de rosuvastatine ou de sels pharmaceutiquement acceptables de celle-ci par le procédé selon la revendication 1, et
b) mélange de la rosuvastatine ou du sel pharmaceutiquement acceptable de celle-ci ainsi préparé avec au moins un excipient pharmaceutiquement acceptable.
